(19) Europäisches Patentamt
European Patent Office
Office européen des brevets

(11) Publication number: 0 252 298 B1

(12) **EUROPEAN PATENT SPECIFICATION**

(45) Date of publication of patent specification: **16.10.91 Bulletin 91/42**

(21) Application number: **87108083.4**

(22) Date of filing: **04.06.87**

(51) Int. Cl.$^5$: **C07C 49/792,** C07C 49/813, C07C 49/84, C07C 205/06, C07C 69/76, C07C 45/54, C07C 255/50, C07C 309/28, C07C 317/14, C07C 323/09, A01N 35/06

(54) **Certain 2-benzoyl-1,3,5-cyclohexanetriones.**

(30) Priority: **09.06.86 US 872067**

(43) Date of publication of application: **13.01.88 Bulletin 88/02**

(45) Publication of the grant of the patent: **16.10.91 Bulletin 91/42**

(84) Designated Contracting States: **AT BE CH DE ES FR GB GR IT LI NL**

(56) References cited:
**EP-A- 0 186 120**
**US-A- 4 227 919**

(73) Proprietor: **STAUFFER CHEMICAL COMPANY**
**Westport Connecticut 06881 (US)**

(72) Inventor: **Carter, Charles Garvie**
**10514 East Wind Way**
**Colombia Maryland 21044 (US)**

(74) Representative: **Kraus, Walter, Dr. et al**
**Patentanwälte Kraus, Weisert & Partner**
**Thomas-Wimmer-Ring 15**
**W-8000 München 22 (DE)**

**EP 0 252 298 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

U.S. Patent 4,202,840 relates to certain 1-hydroxy-2-(alkylketo)-4,4,6,6,-tetramethyl cyclohexene-3,5-diones as herbicides. The compounds have the following strucutural formula

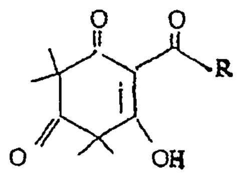

wherein R is alkyl.

This invention relates to 2-benzoyl-1,3,5-cyclohexanetriones and their use as herbicides.

One embodiment of this invention is an herbicidal composition comprising an herbicidally active 2-benzoyl-substituted-1,3,5-cyclohexanetrione and an inert carrier therefor. The 4- and 6-positions of the 1,3,5-cyclohexanetrione moiety are preferably substituted with groups hereinafter defined, most preferably with all methyl groups. The benzoyl moiety can be substituted, preferably with the groups hereinafter recited.

Also embodied within the scope of this invention are novel compounds having the following structural formula

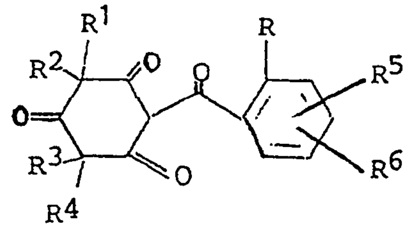

wherein

R is hydrogen, nitro, methyl, methoxy, trifluoromethyl, halogen, or $SO_2CH_3$.

Preferably, R is chlorine, bromine, trifluoromethyl, nitro; more preferably chlorine, nitro, methyl, trifluoromethyl or methylsulfonyl; and

$R^1$ is hydrogen or $C_1$-$C_4$ alkyl, preferably methyl;
$R^2$ is hydrogen or $C_1$-$C_4$ alkyl, preferably methyl; or
$R^1$ and $R^2$ together are $C_2$-$C_5$ alkylene;
$R^3$ is $C_1$-$C_4$ alkyl, preferably methyl;
$R^4$ is $C_1$-$C_4$ alkyl, preferably methyl; or
$R^3$ and $R^4$ together are $C_2$-$C_5$ alkylene;
$R^5$ is hydrogen, 3-$C_1$-$C_4$ alkoxy, 3-nitro or 3-halogen; and
$R^6$ is hydrogen, 4-halogen, 4-trifluoromethyl, 4-$R^bSO_n$ wherein $R^b$ is $C_1$-$C_4$ alkyl or $C_1$-$C_4$ haloalkyl and n is the integer 0 or 2, 4-cyano, 4-C(O)$CH_3$, 4-$SCF_3$ or 4-$SO_2N(CH_3)_2$.

Preferably, $R^5$ and $R^6$ are chlorine, fluorine or bromine, methyl, methoxy, trifluoromethoxy, cyano, nitro and trifluoromethyl.

More preferably $R^5$ is hydrogen, chlorine, fluorine and trifluoromethyl. Most preferably, $R^5$ is hydrogen.

Most preferably $R^6$ is halogen, cyano, trifluoromethyl, or $R^bSO_2$ wherein $R^b$ is $C_1$-$C_4$ alkyl, preferably methyl or $C_1$-$C_4$ haloalkyl, preferably chloromethyl, difluoromethyl or trifluoromethyl.

The term "$C_1$-$C_4$ alkyl" includes methyl, ethyl, n-propyl, isopropyl, n-butyl, sec-butyl, isobutyl and t-butyl. The term "halogen" includes chlorine, bromine, iodine and fluorine. The terms "$C_1$-$C_4$ alkoxy" includes methoxy, ethoxy, n-propoxy, isopropoxy, n-butoxy, sec-butoxy, isobutoxy and t-butoxy. The term "$C_1$-$C_4$ haloalkyl" includes the alkyl groups defined above under $C_1$-$C_4$ alkyl in which one or more hydrogens is replaced by chlorine, bromine, iodine or fluorine.

Salts of the above-described compounds (as defined hereinafter) are included within the scope of the instant invention.

The compounds of this invention can have the following four structural formulae because of tautomerism:

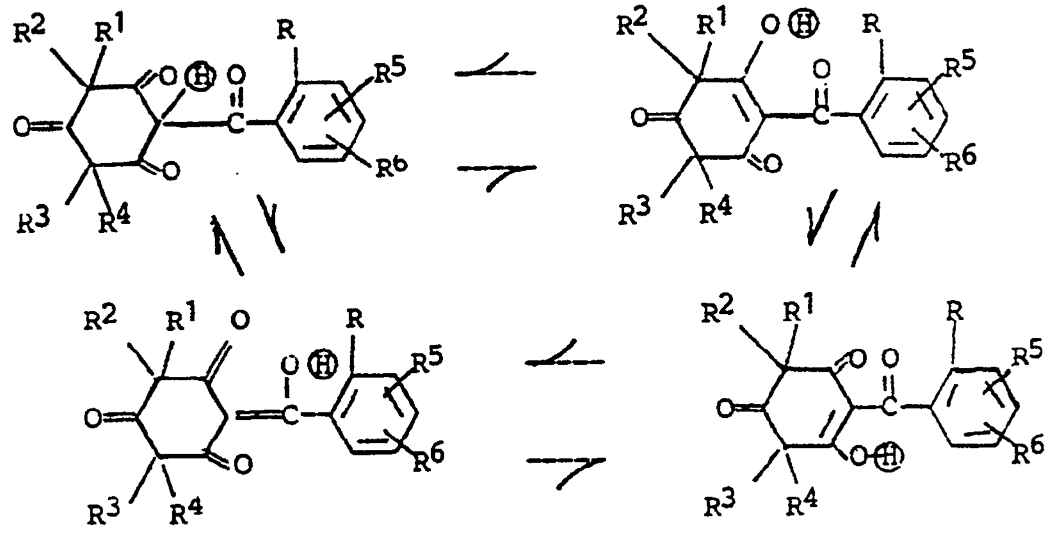

wherein R, $R^1$, $R^2$, $R^3$, $R^4$, $R^5$ and $R^6$ are as defined above

The circled proton on each of the four tautomers is reasonably labile. These protons are acidic and can be removed by reaction with a base to form a salt having an anion of the following four resonance forms:

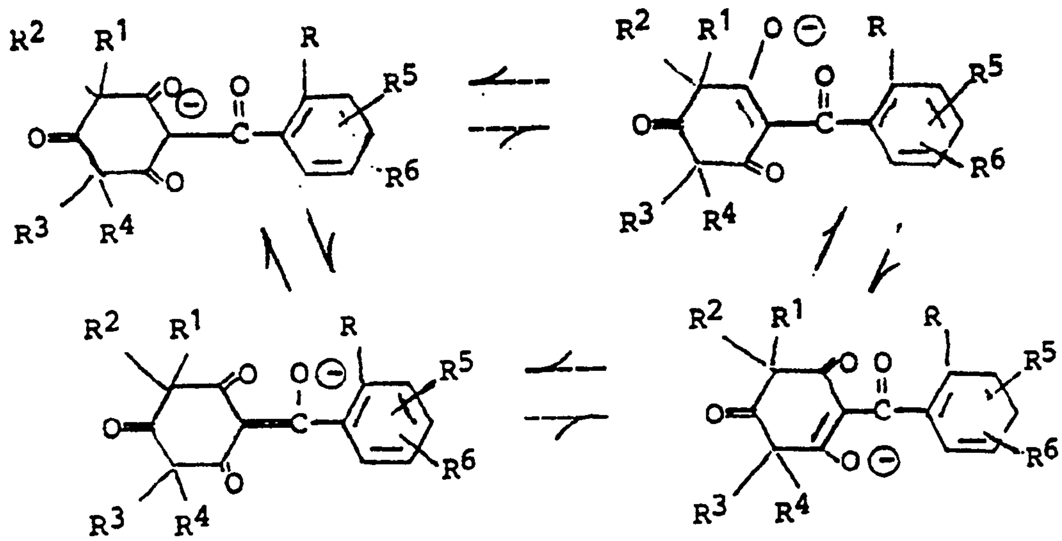

wherein R, $R^1$, $R^2$, $R^3$, $R^4$, $R^5$ and $R^6$ are as defined above.

Examples of cations of these bases are inorganic cations such as alkali metals, e.g. lithium, sodium and potassium; the alkaline earth metals, e.g. calcium and magnesium or ammonium or organic cations such as substituted ammonium, sulfonium, sulfoxonium or phosphonium wherein the substitutents are aliphatic or aromatic groups.

Those skilled in the art will recognize in considering the salts of this invention that varying degrees of association between the anion and cation will exist depending upon the nature of the cation. In some instances with a suitable cation, such as copper, the salt can exist in a chelated form.

The compounds of this invention and their salts are active herbicides of a general type. That is, they are herbicidally effective against a wide range of plant species. The method of controlling undesirable vegetation of the present invention comprises applying an herbicidally effective amount of the above-described compounds or their salts to the area where control is desired.

The compounds of the present invention can be prepared by the following two-step general method.

The process proceeds via the production of an enol ester intermediate as shown in reaction (1). The final product is obtained by rearrangement of the enol ester as shown in reaction (2). The two reactions may be conducted as separate steps by isolation and recovery of the enol ester using conventional techniques prior to conducting step (2), or by addition of a cyanide source to the reaction medium after the formation of the enol ester, or in one step by inclusion of the cyanide source at the start of reaction (1).

1)

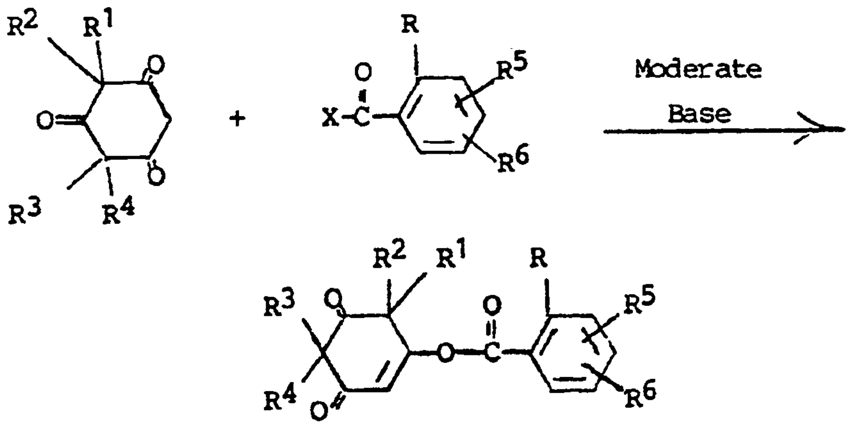


wherein R through $R^6$ and moderate base are as defined and X is halogen, preferably chlorine, $C_1$-$C_4$ alkyl-C(O)-O-, $C_1$-$C_4$ alkoxy-C(O)-O- or

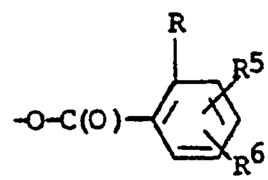

wherein R, $R^5$ and $R^6$ in this portion of the molecule are identical with those in the reactant shown above and the moderate base is as defined, preferably tri-$C_1$-$C_6$ alkylamine, alkali metal carbonate or alkali metal phosphate.

Generally, in step (1) mole amounts of the trione and substituted benzoyl reactant are used, along with a mole or excess of the base. The two reactants are combined in an organic solvent such as methylene chloride, toluene, ethyl acetate or dimethylformamide. The base or benzoyl reactant preferably is added to the reaction mixture with cooling. The mixture is stirred at 0°C-50°C until the reaction is substantially complete.

2)

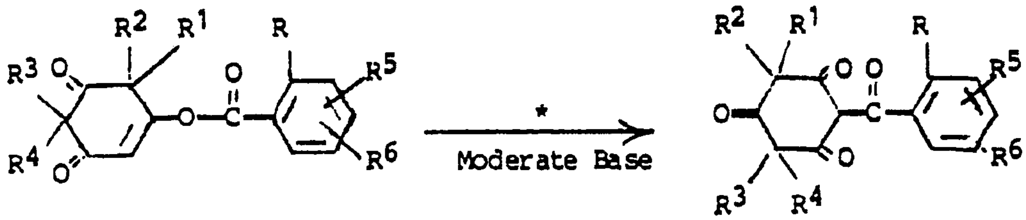


* = Cyanide source.

wherein the moderate base and R through $R^6$ are as defined above.

Generally, in step (2) a mole of the enol ester intermediate is reacted with 1 to 4 moles of the moderate base, preferably about 2 moles of moderate base and from 0.01 mole to about 0.5 mole or higher, preferably about 0.1 mole of the cyanide source (e.g., potassium cyanide or acetone cyanohydrin). The mixture is stirred in a reaction pot until the rearrangement is substantially complete at a temperature below 50°C, preferably about 20°C to about 40°C, and the desired product is recovered by conventional techniques.

The term "cyanide source" refers to a substance or substances which under the rearrangement conditions consists of or generates hydrogen cyanide and/or cyanide anion.

The process is conducted in the presence of a catalytic amount of a source of cyanide anion and/or hydrogen cyanide, together with a molar excess, with respect to the enol ester, of a moderate base.

Preferred cyanide sources are alkali metal cyanides such as sodium and potassium cyanide; cyanohydrins of methyl alkyl ketones having from 1-4 carbon atoms in the alkyl groups, such as acetone or methyl isobutyl ketone cyanohydrins; cyanohydrins of benzaldehyde or of $C_2$-$C_5$ aliphatic aldehydes such as acetaldehyde, propionaldehyde, etc., cyanohydrins; zinc cyanide; tri(lower alkyl) silyl cyanides, notably trimethyl silyl cyanide; and hydrogen cyanide itself. Hydrogen cyanide is considered most advantageous as it produces relatively rapid

reaction and is inexpensive. Among cyanohydrins the preferred cyanide source is acetone cyanohydrin.

The cyanide source is used in an amount up to about 50 mole percent based on the enol ester. It may be used in as little as about 1 mole percent to produce an acceptable rate of reaction at about 40°C on a small scale. Larger scale reactions give more reproductible results with slightly higher catalyst levels of about 2 mole percent. Generally about 1-10 mole % of the cyanide source is preferred.

The process is conducted with a molar excess, with respect to the enol ester, of a moderate base. By the term "moderate base" is meant a substance which acts as a base yet whose strength or activity as a base lies between that of strong bases such as hydroxides (which could cause hydrolysis of the enol ester) and that of weak bases such as bicarbonates (which would not function effectively). Moderate bases suitable for use in this embodiment include both organic bases, e.g., trialkylamines such as triethylamine and inorganic bases such as alkali metal carbonates and phosphates. Suitable inorganic bases include potassium carbonate and trisodium phosphate.

The base is used in an amount of from about 1 to about 4 moles per mole of enol ester, preferably about 2 moles per mole.

When the cyanide source is an alkali metal cyanide, particularly potassium cyanide, a phase transfer catalyst may be included in the reaction. Particularly suitable phase transfer catalysts are the crown ethers.

A number of different solvents are useful in this process, depending on the nature of the acid halide or the acylated product. A preferred solvent for this reaction is 1,2-dichloroethane. Other solvents which may be employed, depending on the reactants or products include toluene, acetonitrile, methylene chloride, ethyl acetate, dimethylformamide, and methyl isobutyl ketone (MIBK).

In general, depending on the nature of the reactants and the cyanide source, the rearrangement may be conducted at temperature up to about 50°C.

The above described substituted benzoyl chlorides can be prepared from the corresponding substituted benzoic acids according to the teaching of Reagents for Organic Synthesis, Vol. I, L.F. Fieser and M. Fieser, pop. 767-769 (1967).

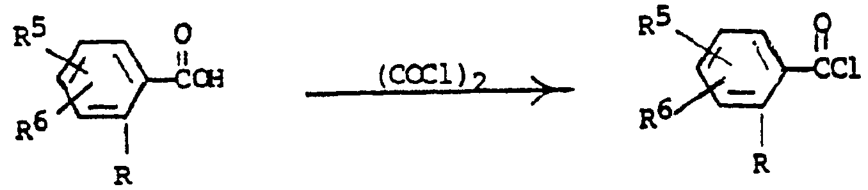


wherein R, $R^5$ and $R^6$ are as previously defined.

The substituted benzoic acids can be prepared by a wide variety of general methods according to the teaching of The Chemistry of Carboxylic Acids and Esters, S. Patai, editor, J. Wiley and Sons, New York, N.Y. (1969) and Survey of Organic Synthesis, C.A. Buehler and D.F. Pearson, J. Wiley and Sons, (1970).

The following are three representative examples of the methods described therein.

a) 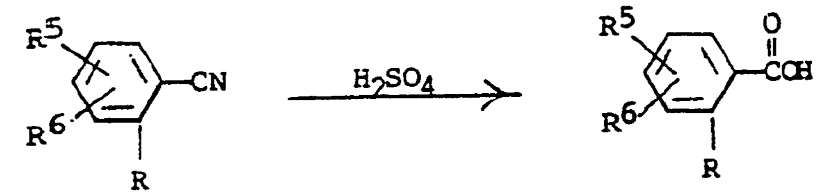


wherein R, $R^5$ and $R^6$ are as previously defined.

In reaction (a) the substituted benzonitrile is heated to reflux in aqueous sulfuric acid for several hours. The mixture is cooled and the reaction product is isolated by conventional techniques.

b) 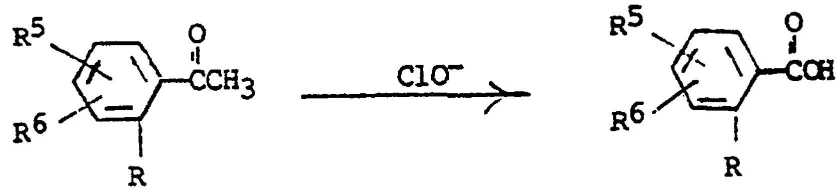


wherein R, $R^5$ and $R^6$ are as previously defined.

In reaction (b) the substituted acetophenone is heated to reflux for several hours in an aqueous hypochlorite solution. The mixture is cooled and the reaction product is isolated by conventional techniques.

c) 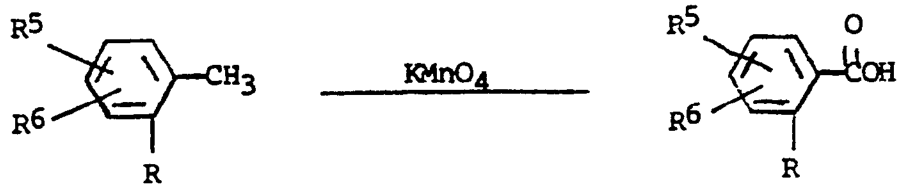


wherein R, $R^5$ and $R^6$ are as previously defined.

In reaction (c) the substituted toluene is heated to reflux in an aqueous solution of potassium permanganate for several hours. The solution is then filtered and the reaction product is isolated by conventional techniques.

The following example teaches the synthesis of a representative compound of this invention.

EXAMPLE 1

2-(2'-Nitro-4'-chlorobenzoyl)-4,4,6,6-tetramethyl-1,3,5-cyclohexanetrione

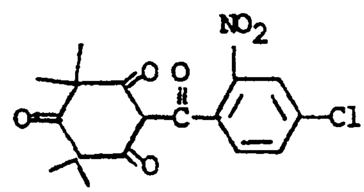

2-Nitro-4-chlorobenzoyl chloride (2.2 g, 10 mmol) and 4,4,6,6-tetramethyl-1,3,5-cyclobexanetrione (1.8 g, 10 mmol) were dissolved in methylene chloride. Triethylamine was added and the resulting solution stirred at room temperature for 30 minutes. The solution was washed with 1 normal hydrochloric acid (1N HCl), and saturated sodium chloride (brine), dried over anhydrous magnesium sulfate ($MgSO_4$) and concentrated under vacuum. The residue was dissolved in 20 ml acetonitrile. Triethylamine (5 ml, 3.5 equivalents) and acetone cyanohydrin (0.5 g, 0.6 equivalent) were added and the mixture stirred at room temperature for 4 hours. After dilution with ether, the solution was washed with 1N HCl and extracted with 5% $K_2CO_3$. The basic extract was acidified with HCl and extracted with ether. The ether extract was washed with brine, dried over $MgSO_4$ and concentrated under vacuum, yielding 2.2 g of crude product. This was recrystallized from benzene to remove syncarpic acid still present. Concentration of the mother liquor under vacuum gave 1.7 g of the desired product as an oil, which solidifed on standing (m.p. 76-82°C). It was identified as such by nuclear magnetic resonance spectroscopy, infrared spectroscopy and mass spectroscopy.

The following is a table of certain selected compounds that are preparable according to the procedure described hereto. Compounds numbers are assigned to each compound and are used throughout the remainder of the application.

TABLE I

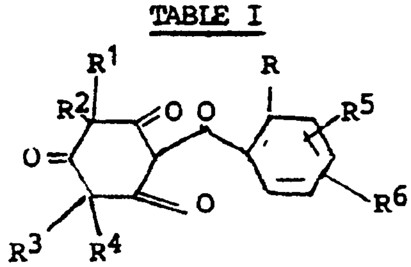

| Comp. No. | R | $R^1$ | $R^2$ | $R^3$ | $R^4$ | $R^5$ | $R^6$ | m.p. °C |
|---|---|---|---|---|---|---|---|---|
| 1 | $NO_2$ | $CH_3$ | $CH_3$ | $CH_3$ | $CH_3$ | H | H | 112-117 |
| 2[a] | $NO_2$ | $CH_3$ | $CH_3$ | $CH_3$ | $CH_3$ | H | 4-Cl | 76-82 |
| 3 | Cl | $CH_3$ | $CH_3$ | $CH_3$ | $CH_3$ | H | 4-$SO_2CH_3$ | 176-179° |
| 4 | $CH_3$ | $CH_3$ | $CH_3$ | $CH_3$ | $CH_3$ | H | 4-$SO_2C_2H_5$ | gum |
| 5 | $CF_3$ | $CH_3$ | $CH_3$ | $CH_3$ | $CH_3$ | H | 4-$CF_3$ | viscous oil |
| 6 | $NO_2$ | $CH_3$ | $CH_3$ | $CH_3$ | $CH_3$ | H | 4-$SO_2CH_2Cl$ | oil |
| 7 | Cl | $CH_3$ | $CH_3$ | $CH_3$ | $CH_3$ | H | 4-F | gum |
| 8 | Cl | $CH_3$ | $CH_3$ | $CH_3$ | $CH_3$ | 3-$OC_2H_5$ | 4-$SO_2C_2H_5$ | gum |
| 9 | $NO_2$ | $CH_3$ | $CH_3$ | $CH_3$ | $CH_3$ | H | 4-$SO_2CH_3$ | gum |
| 10 | Cl | $CH_3$ | $CH_3$ | $CH_3$ | $CH_3$ | H | 4-$SCH_3$ | gum |
| 11 | $CH_3$ | $CH_3$ | $CH_3$ | $CH_3$ | $CH_3$ | 3-$NO_2$ | H | gum |
| 12 | I | $CH_3$ | $CH_3$ | $CH_3$ | $CH_3$ | H | H | gum |
| 13 | $OCH_3$ | $CH_3$ | $CH_3$ | $CH_3$ | $CH_3$ | H | 4-Cl | gum |
| 14 | Cl | $CH_3$ | $CH_3$ | $CH_3$ | $CH_3$ | 3-$OCH_3$ | 4-Br | gum |
| 15 | $NO_2$ | $CH_3$ | $CH_3$ | $CH_3$ | $CH_3$ | H | 4-$SO_2nC_3H_7$ | gum |
| 16 | $NO_2$ | $CH_3$ | $CH_3$ | $CH_3$ | $CH_3$ | H | 4-$SO_2N(CH_3)_2$ | gum |
| 17 | $NO_2$ | $CH_3$ | $CH_3$ | $CH_3$ | $CH_3$ | H | 4-CN | 148-150° |
| 18 | $SO_2CH_3$ | $CH_3$ | $CH_3$ | $CH_3$ | $CH_3$ | H | H | gum |
| 19 | $NO_2$ | $CH_3$ | $CH_3$ | $CH_3$ | $CH_3$ | H | 4-$CF_3$ | gum |
| 20 | H | $CH_3$ | $CH_3$ | $CH_3$ | $CH_3$ | H | 4-Cl | gum |
| 21 | H | $CH_3$ | $CH_3$ | $CH_3$ | $CH_3$ | H | 4-$SCF_3$ | gum |
| 22 | H | $CH_3$ | $CH_3$ | $CH_3$ | $CH_3$ | H | 4-$OCF_3$ | gum |
| 23 | Cl | $CH_3$ | $CH_3$ | $CH_3$ | $CH_3$ | 3-Cl | 4-$SO_2C_2H_5$ | gum |
| 24 | H | $CH_3$ | $CH_3$ | $CH_3$ | $CH_3$ | H | 4-C(O)$CH_3$ | gum |
| 25 | H | $CH_3$ | $CH_3$ | $CH_3$ | $CH_3$ | H | 4-$SO_2$-$CH_3$ | 129-131 |
| 26 | H | $CH_3$ | $CH_3$ | $CH_3$ | $CH_3$ | H | 4-$SCH_3$ | 89-92 |
| 27 | H | $CH_3$ | $CH_3$ | $CH_3$ | $CH_3$ | H | 4-$CF_3$ | 115-125 |
| 28 | H | $CH_3$ | $CH_3$ | $CH_3$ | $CH_3$ | H | 4-CN | 87-95 |

(a) prepared in Example 1.

Herbicidal Screening Tests

A previously mentioned, the herein described compounds produced in the above-described manner are phytotoxic compounds which are useful and valuable in controlling various plant species. Selected compounds of this invention were tested as herbicides in the following manner.

Pre-emergence herbicide test. On the day preceding treatment, seeds of seven different weed species are planted in loamy sand oil in individual rows using one species per row across the width of a flat. The seeds used are green foxtail (FT) (Setaria viridis), watergrass (WG) (Echinochloa crusgalli), wild oat (WO) (Avena fatua), annual morigglory (AMG) (Ipomoea lacunosa), velvetleaf (VL) (Abutilon theophrasti), Indian mustard (MD) (Brassica juncea), and yellow nutsedge (YNG) (Cyperus esculentus). Ample seeds are planted to give about 20 to 40 seedlings per row, after emergence, depending upon the size of the plants.

Using an analytical balance, 600 milligrams (mg) of the compound to be tested are weighed out on a piece of glassine weighing paper. The paper and compound are placed in a 60 milliliter (ml) wide-mouth clear bottle and dissolved in 45 ml of acetone or substituted solvent. Eighteen ml of this solution are transferred to a 60 ml wide-mouth clear bottle and diluted with 22 ml of a water and acetone mixture (19:1) containing enough polyoxyethylene sorbitan monolaurate emulsifier to give a final solution of 0.5% (v/v). The solution is then sprayed on a seeded flat on a linear spray table calibrated to deliver 80 gallons per acre (748 L/ha). The application rate is 4 lb/acre (4.48 Kg/ha).

After treatment, the flats are placed in the greenhouse at a temperature of 70 to 80°F and watered by sprinkling. Two weeks after treatment, the degree of injury or control is determined by comparison with untreated check plants of the same age. The injury rating from 0 to 100% is recorded for each species as percent control with 0% representing no injury and 100% representing complete control.

The results of the tests are shown in the following Table II.

TABLE II

Pre-Emergence Herbicidal Activity
Application Rate — 4.48 kg/ha

| Cmpd. No. | FT | WG | WO | AMG | VL | MD | YNG |
|---|---|---|---|---|---|---|---|
| 1 | 100 | 100 | 90 | 100 | 100 | 100 | 80 |
| 2 | 100 | 100 | 90 | 100 | 100 | 100 | 80 |
| 3 | 100 | 100 | 90 | 100 | 100 | 100 | 80 |
| 4 | 100 | 100 | 90 | 100 | 100 | 100 | 80 |
| 5 | 100 | 100 | 90 | 100 | 100 | 100 | 80 |
| 6* | 100 | 100 | 100 | 100 | 100 | - | 20 |
| 7 | 100 | 100 | 80 | 100 | 100 | 100 | 80 |
| 8 | 85 | 100 | 80 | 100 | 100 | 100 | 80 |
| 9 | 100 | 100 | 80 | 100 | 100 | 100 | 80 |
| 10 | 100 | 100 | 80 | 100 | 100 | 100 | 80 |
| 11 | 100 | 100 | 80 | 100 | 100 | 100 | 80 |
| 12 | 100 | 100 | 80 | 90 | 100 | 100 | 0 |
| 13 | 100 | 100 | 70 | 100 | 100 | 100 | 0 |
| 14 | 100 | 100 | 75 | 100 | 100 | 100 | 90 |
| 15 | 100 | 100 | 80 | 100 | 100 | 100 | 90 |
| 16 | 100 | 100 | 90 | 100 | 100 | 100 | 80 |
| 17 | 100 | 100 | 80 | 100 | 100 | 100 | 80 |
| 18 | 100 | 100 | 90 | 85 | 100 | 100 | 80 |
| 19 | 100 | 100 | 100 | 100 | 100 | 100 | 80 |
| 20 | 70 | 95 | 10 | 100 | 100 | 100 | 0 |
| 21 | 100 | 100 | 100 | 100 | 100 | 100 | 80 |
| 22 | 100 | 100 | 90 | 100 | 100 | 100 | 80 |
| 23 | 100 | 100 | 100 | 100 | 100 | 100 | 80 |
| 24 | 0 | 80 | 0 | 25 | 80 | 70 | 0 |
| 25 | 100 | 100 | 80 | 100 | 100 | 100 | 80 |
| 26 | 100 | 100 | 100 | 80 | 100 | 100 | 80 |
| 27 | 100 | 100 | 100 | 100 | 100 | 100 | 80 |
| 28 | 100 | 100 | 90 | 100 | 100 | 100 | 80 |

A blank (-) indicates that the weed was not tested.

* = Tested at 0.56 kg/ha.

Post-Emergence Herbicide Test: This test is conducted in an identical manner to the testing procedure for the pre-emergence herbicide test, except the seeds of the seven different weed species are planted 10-12 days before treatment. Also, watering of the treated flats is confined to the soil surface and not to the foliage of the sprouted plants.

The resluts of the post-emergence herbicide test are reported in Table III.

TABLE III

Post-Emergence Herbicidal Activity
Application Rate — 4.48 kg/ha

| Cmpd. No. | FT | WG | WO | AMG | VL | MD | YNG |
|---|---|---|---|---|---|---|---|
| 1 | 100 | 95 | 90 | 90 | 100 | 100 | 80 |
| 2 | 100 | 100 | 100 | 100 | 100 | 100 | 80 |
| 3 | 100 | 100 | 95 | 100 | 100 | 100 | 90 |
| 4 | 100 | 100 | 100 | 100 | 100 | 100 | 90 |
| 5 | 100 | 100 | 100 | 100 | 100 | 100 | 100 |
| 6* | 10 | 80 | 50 | 75 | 40 | - | 0 |
| 7 | 70 | 75 | 70 | 90 | 90 | 80 | 80 |
| 8 | 50 | 80 | 70 | 90 | 90 | 80 | 30 |
| 9 | 70 | 80 | 70 | 90 | 90 | 80 | 30 |
| 10 | 75 | 80 | 70 | 80 | 60 | 50 | 70 |
| 11 | 70 | 80 | 70 | 80 | 80 | 80 | 80 |
| 12 | 100 | 75 | 90 | 75 | 80 | 80 | 80 |
| 13 | 60 | 70 | 70 | 100 | 100 | 100 | 80 |
| 14 | 20 | 70 | 40 | 60 | 80 | 80 | 70 |
| 15 | 90 | 70 | 70 | 75 | 80 | 90 | 70 |
| 16 | 50 | 70 | 80 | 60 | 80 | 90 | 70 |
| 17 | 90 | 60 | 70 | 65 | 80 | 80 | 80 |
| 18 | 30 | 50 | 80 | 95 | 95 | 80 | 70 |
| 19 | 100 | 100 | 95 | 90 | 100 | 100 | 80 |
| 20 | 10 | 40 | 5 | 70 | 80 | 90 | 70 |
| 21 | 95 | 90 | 95 | 95 | 100 | 100 | 70 |
| 22 | 80 | 90 | 90 | 80 | 100 | 100 | 70 |
| 23 | 90 | 80 | 80 | 80 | 90 | 80 | 30 |
| 24 | 0 | 30 | 0 | 40 | 80 | 20 | 0 |
| 25 | 0 | 20 | 0 | 10 | 10 | 20 | 0 |
| 26 | 80 | 70 | 70 | 80 | 80 | 80 | 70 |
| 27 | 90 | 90 | 90 | 80 | 80 | 90 | 60 |
| 28 | 70 | 65 | 50 | 80 | 80 | 80 | 70 |

A blank (-) indicates the weed was not tested.

* = Tested at 0.56 kg/ha.

The compounds of the present invention and their salts are useful as herbicides and can be applied in a variety of ways at various concentrations. In practice, the compounds or salts are formulated into herbicidal

compositions, by admixture, in herbicidally effective amounts, with the adjuvants and carriers normally employed for facilitating the dispersion of active ingredients for agricultural applications, recognizing the fact that the formulation and mode of application of a toxicant may affect the activity of the materials in a given application. Thus, these active herbicidal compounds or salts can be formulated as granules of relatively large particle size, as wettable powders, as emulsifiable concentrates, as powdery dusts, as flowables, as solutions or as any of several other known types of formulations, depending upon the desired mode of application. These formulations may contain as little as about 0.5% to as much as about 95% or more try weight of active ingredient. A herbicidally effective amount depends upon the nature of the seeds or plants to be controlled and the rate of application varies from about 0.01 to approximately 10 pounds per acre, preferably from about 0.02 to about 4 pounds per acre.

Wettable powders are in the form of finely divided particles which disperse readily in water or other dispersants. The wettable powder is ultimately applied to the soil either as a dry dust or as a dispersion in water or other liquid. Typical carriers for wettable powders include fuller's earth, kaolin clays, silicas and other readily wet organic or inorganic diluents. Wettable powders normally are prepared to contain about 5% to about 95% of the active ingredient and usually also contain a small amount of wetting, dispersing or emulsifying agent to facilitate wetting and dispersion.

Emulsifiable concentrates are homogeneous liquid compositions which are dispersible in water or other dispersant, and may consist entirely of the active compound or salt with a liquid or solid emulsifying agent, or may also contain a liquid carrier, such as xylene, heavy aromatic naphthal, isophorone and other non-volatile organic solvents. For herbicidal application, these concentrates are dispersed in water or other liquid carrier and normally applied as a spray to the area to be treated. The percentage by weight of the essential active ingredient may vary according to the manner in which the composition is to be applied, but in general comprises about 0.5% to 95% of active ingredient by weight of the herbicidal composition.

Granular formulations wherein the toxicant is carried on relatively coarse particles, are usually applied without dilution to the area in which suppression of vegetation is desired. Typical carriers for granular formulations include sand, fuller's earth, attapulgite clay, bentonite clays, montmorillonite clay, vermiculite, perlite and other organic or inorganic materials which absorb or which may be coated with the toxicant. Granular formulations normally are prepared to contain about 5% to about 25% of active ingredients which may include surface-active agents such heavy aromatic naphthas, kerosene or other petroleum fractions, or vegetable oils; and/or stickers such as destrins, glue or synthetic resins.

Typical wetting, dispersing or emulsifying agents used in agricultural formulations include, for example, the alkyl and alkylaryl sulfonates and sulfates and their salts; polyhydric alcohols; polyethoxylated alcohols; esters and fatty amines; and others types of surface-active agents, many of which are available in commerce. The surface-active agent, when used, normally comprises from 0.1% to 15% by weight of the herbicidal composition.

Dusts, which are free-flowing admixtures of the active ingredient with finely divided solids such as talc, clays, flours and other organic and inorganic solids which act as dispersants and carriers for the toxicant, are useful formulations for soil-incorporating application.

Pastes, which are homogeneous suspensions of a finely divided solid toxicant in a liquid carrier such as water or oil, are employed for specific purposes. These formulations normally contain about 5% to about 95% of active ingredient by weight, and may also contain small amounts of a wetting, despersing or emulsifying agent to facilitate dispersion. For application, the pastes are normally diluted and applied as a spray to the area to be affected.

Other useful formulations for herbicidal applications include simple solutions of the active ingredient in a dispersant in which it is completely soluble at the desired concentration, such as acetone, alkylated naphthalenes, xylene and other organic solvents. Pressurized sprays, typically aerosols, wherein the active ingredient is dispersed in finely-divided form as a result of vaporization of a low boiling dispersant solvent carrier, such as the Freons, may also be used.

The phytotoxic compositions of this invention can be applied to the plants in the conventional manner. Thus, the dust and liquid compositions can be applied to the plant by the use of power-dusters, boom and hand sprayers and spray dusters. The compositions can also be applied from airplanes as a dust or a spray or by rope wick applications because they are effective in very low dosages. In order to modify or control growth of germinating seeds or emerging seedlings, as a typical example, the dust and liquid compositions can be applied to the soil according to conventional methods and can be distributed in the soil to a depth of at least 1/2 inch below the soil surface. It is not necessary that the phytotoxic compositions be mechanically admixed with the soil particles since these compositions can also be applied merely by spraying or sprinkling the surface of the soil. The phytotoxic compositions of this invention can also be applied by additioin to irrigation water supplied to the field to be treated. This method of application permits the penetration of the compositions into the soil as the water is absorbed therein. Dust compositions, granular compositions or liquid formulations applied to

the surface of the soil can be distributed below the surface of the soil by conventional means such as discing, dragging or mixing operations. In the following examples the herbicidal compound can be substituted with the herbicidal salt of the compound.

EMULSIFIABLE CONCENTRATE FORMULATIONS

| General Formula with Ranges | | Specific Formula | |
|---|---|---|---|
| Herbicidal compound | 5-55 | herbicidal compound | 24 |
| surfactant(s) | 5-25 | proprietary blend of oil-soluble sulfonates and polyoxyethylene ethers | 10 |
| solvent(s) | 20-90 | polar solvent | 27 |
| | | petroleum hydrocarbon | 39 |
| | 100% | | 100% |

WETTABLE POWDER FORMULATIONS

| | | | |
|---|---|---|---|
| herbicidal compound | 3-90 | herbicidal compound | 80 |
| wetting agent | 0.5-2 | sodium dialkyl naphthalene sulfonate | 0.5 |
| dispersing agent | 1-8 | sodium lignosulfonate | 7 |
| diluent(s) | 8.5-87 | attapulgite clay | 12.5 |
| | 100% | | 100% |

EXTRUDED GRANULAR FORMULATIONS

| | | | |
|---|---|---|---|
| herbicidal compound | 1-20 | herbicidal compound | 10 |
| binding agent | 0-10 | lignin sulfonate | 5 |
| diluent(s) | 70-99 | calcium carbonate | 85 |
| | 100% | | 100% |

FLOWABLE FORMULATIONS.

| | | | |
|---|---|---|---|
| herbicidal compound | 20-70 | herbicidal compound | 45 |
| surfactant(s) | 1-10 | polyoxyethylene ether | 5 |
| suspending agent(s) | 0.05-1 | attagel | 0.05 |
| antifreeze agent | 1-10 | propylene glycol | 10 |
| antimicrobial agent | 1-10 | 1,2-benzisothiazoline-3-one | 0.03 |
| antifoam agent | 0.1-1 | silicone defoamer | 0.02 |
| solvent | 7.95-77.85 | water | 39.9 |
| | 100% | | 100% |

When salts are used as the active ingredient in the herbicidal compositions of this invention it is recommended to use salts that are agriculturally acceptable.

The phytotoxic compositions of this invention can also contain other additives, for example, fertilizers, other herbicides and other pesticides, used as adjuvant or in combination with any of the above-described adjuvants. Fertilizers useful in combination with the active ingredients include, for example, ammonium nitrate, urea and superphosphate.

## Claims

### Claims for the following Contracting States : BE, CH, DE, FR, GB, GR, IT, LI and NL

1. Compounds of the formula

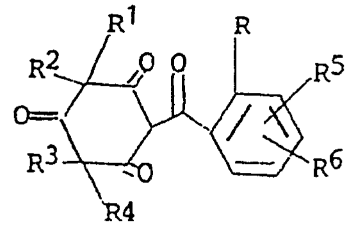

wherein

R is hydrogen, nitro, methyl, methoxy, trifluoromethyl, halogen or $SO_2CH_3$;
$R^1$ is hydrogen or $C_1$-$C_4$ alkyl;
$R^2$ is hydrogen or $C_1$-$C_4$ alkyl; or
$R^1$ and $R^2$ together are $C_2$-$C_5$ alkylene;
$R^3$ is $C_1$-$C_4$ alkyl;
$R^4$ is $C_1$-$C_4$ alkyl; or
$R^3$ and $R^4$ together are $C_2$-$C_5$ alkylene;
$R^5$ is hydrogen, 3-$C_1$-$C_4$ alkoxy, 3-nitro or 3-halogen; and
$R^6$ is hydrogen 4-halogen, 4-trifluoromethyl, 4-$R^bSO_n$, wherein $R^b$ is $C_1$-$C_4$ alkyl or- $C_1$-$C_4$ haloalkyl and n is the integer 0 or 2, 4-cyano, 4-C(O)$CH_3$, 4-$SCF_3$ or: 4-$SO_2N(CH_3)_2$,

and their salts.

2. The compounds of Claim 1 wherein R is chlorine, hydrogen, bromine, methyl, methoxy, nitro, trifluoromethyl or methylsulfonyl; $R^1$, $R^2$, $R^3$ and $R^4$ are methyl; $R^5$ and $R^6$ independently are (1) hydrogen; (2) halogen; (3) $C_1$-$C_4$ alkyl; (4) $C_1$-$C_4$ alkoxy; (5) cyano; (6) nitro; (7) $C_1$-$C_4$ haloalkyl; (8) $R^bSO_n$- wherein n is the integer 0 or 2; and $R^b$ is (a) $C_1$-$C_4$ alkyl; (b) $C_1$-$C_4$ alkyl substituted with halogen; (9) 4-$SO_2N(CH_3)_2$.

3. The compounds of Claim 2 wherein $R^5$ is hydrogen and $R^6$ is hydrogen, chlorine, bromine, fluorine, cyano, trifluoromethyl or $R^bSO_2$ wherein $R^b$ is $C_1$-$C_4$ alkyl or $C_1$-$C_4$ haloalkyl.

4. The compounds of Claims 2 or 3 wherein $R^5$ is hydrogen.

5. The method of controlling undesirable vegetation comprising applying to the area where control is desired, an herbicidally effective amount of a compound described in Claims 1, 2, 3 or 4.

6. An herbicidal composition comprising an herbicidally active 2-benzoyl-substituted-1,3,5-cyclohexanetrione or their salts as described in Claims 1, 2, 3 or 4 and an inert carrier therefor.

7. The method of controlling undesirable vegetation comprising applying to the area where control is desired, an herbicidal composition comprising an herbicidally active 2-benzoyl-substituted-1,3,5-cyclohexanetrione or its salt as described in Claim 6 and an inert carrier therefor.

8. An intermediate compound of the structural formula

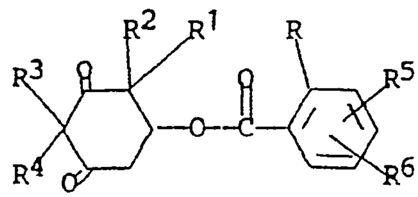

wherein

R is hydrogen, nitro, methyl, methoxy, trifluoromethyl, halogen or $SO_2CH_3$;
$R^1$ is hydrogen or $C_1$-$C_4$ alkyl;
$R^2$ is hydrogen or $C_1$-$C_4$ alkyl; or
$R^1$ and $R^2$ together are $C_2$-$C_5$ alkylene;
$R^3$ is $C_1$-$C_4$ alkyl;
$R^4$ is $C_1$-$C_4$ alkyl; or:
$R^3$ and $R^4$ together are $C_2$-$C_5$ alkylene;
$R^5$ is hydrogen, 3-$C_1$-$C_4$ alkoxy, 3-nitro or 3-halogen; and
$R^6$ is hydrogen, 4-halogen, 4-trifluoromethyl, 4-$R^bSO_n$ wherein $R^b$ is $C_1$-$C_4$ alkyl or $C_1$-$C_4$ haloalkyl and n is the integer 0 or 2, 4-cyano, 4-C(O)$CH_3$, 4-$SCF_3$ or 4-$SO_2N(CH_3)_2$.

9. A process for preparing an herbicidal compound having the structural formula

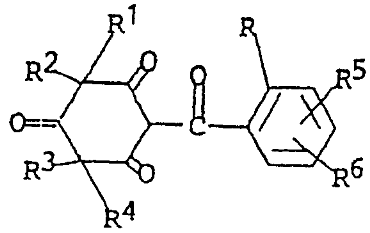

wherein

R is hydrogen, nitro, methyl, methoxy, trifluoromethyl, halogen or $SO_2CH_3$;

$R^1$ is hydrogen or $C_1$-$C_4$ alkyl;

$R^2$ is hydrogen or $C_1$-$C_4$ alkyl; or

$R^1$ and $R^2$ together: are $C_2$-$C_5$ alkylene;

$R^3$ is $C_1$-$C_4$ alkyl;

$R^4$ is $C_1$-$C_4$ alkyl; or

$R^3$ and $R^4$ together are $C_2$-$C_5$ alkylene;

$R^5$ is hydrogen, 3-$C_1$-$C_4$ alkoxy, 3-nitro or 3-halogen; and

$R^6$ is hydrogen, 4-halogen, 4-trifluoromethyl, 4-$R^bSO_n$ wherein $R^b$ is $C_1$-$C_4$ alkyl or $C_1$-$C_4$ haloalkyl and n is the integer 0 or 2, 4-cyano, 4-$C(O)CH_3$, 4-$SCF_3$ or 4-$SO_2N(CH_3)_2$,

and their salts;

comprising the steps of 1) reacting a mole amount of a trione of the structural formula

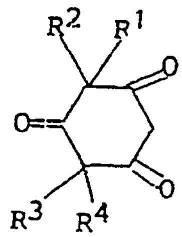

wherein $R^1$ to $R^4$ are as defined with a mole amount of a substituted benzoyl reactant of the structural formula

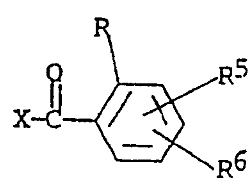

wherein R, $R^5$ and $R^6$ are as defined and X is halogen, $C_1$-$C_4$ alkyl-C(O)-O-, $C_1$-$C_4$ alkoxy-C(O)-O- or

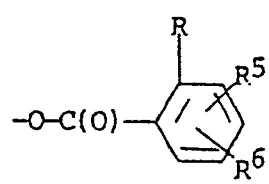

wherein R, $R^5$ and $R^6$ in this portion of the molecule are identical with those in the reactant shown above along with a mole amount or more of a moderate base to form an enol ester of the structural formula

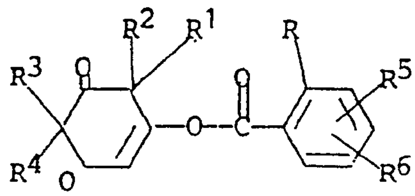

wherein R to $R^6$ are as defined and in step 2) reacting a mole amount of the enol ester intermediate with 1 to 4 moles of a moderate base and from 0.01 mole to 0.5 mole or higher of a cyanide source to form the desired herbicidal compound.

10. The process of Claim 9 wherein X is halogen, the moderate base is tri-$C_1$-$C_6$ alkylamine, pyridine, alkali metal carbonate or alkali metal phosphate and the cyanide source alkali metal cyanide, cyanohydrins of methyl $C_1$-$C_4$-alkyl ketones, cyanohydrins of benzaldehyde or $C_2$-$C_5$ aliphatic aldehydes; cyanohydrins, zinc cyanide; tri (lower alkyl) silyl cyanides or hydrogen cyanide.

11. The process of Claim 10 wherein X is chlorine, the moderate base is tri-$C_1$-$C_6$ alkylamine, pyridine, sodium carbonate or sodium phosphate and the cyanide source is potassium cyanide, acetone cyanohydrin or hydrogen cyanide.

## Claims for the following Contracting States : AT and ES

1. A process for preparing a herbicidal compound having the structural formula

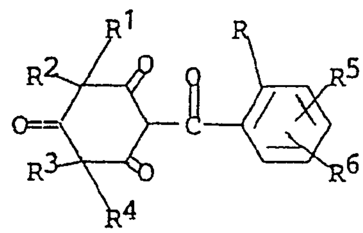

wherein

R is hydrogen, nitro, methyl, methoxy, trifluoromethyl, halogen or $SO_2CH_3$;
$R^1$ is hydrogen or $C_1$-$C_4$ alkyl;
$R^2$ is hydrogen or $C_1$-$C_4$ alkyl; or
$R^1$ and $R^2$ together are $C_2$-$C_5$ alkylene;
$R^3$ is $C_1$-$C_4$ alkyl;
$R^4$ is $C_1$-$C_4$ alkyl; or
$R^3$ and $R^4$ together are $C_2$-$C_5$ alkylene;
$R^5$ is hydrogen, 3-$C_1$-$C_4$ alkoxy, 3-nitro or 3-halogen; and
$R^6$ is hydrogen, 4-halogen, 4-trifluoromethyl, 4-$R^bSO_n$ wherein $R^b$ is $C_1$-$C_4$ alkyl or $C_1$-$C_4$ haloalkyl and n is the integer 0 or 2, 4-cyano, 4-C(O)$CH_3$, 4-$SCF_3$ or 4-$SO_2N(CH_3)_2$,

and their salts;
comprising the steps of 1) reacting a mole amount of a trione of the structural formula

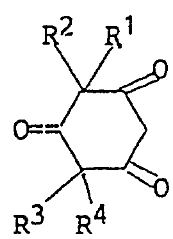

wherein $R^1$ to $R^4$ are as defined with a mole amount of a substituted benzoyl reactant of the structural formula

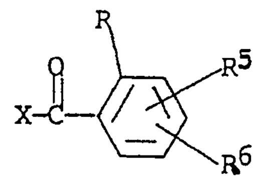

wherein R, $R^5$ and $R^6$ are as defined and X is halogen, $C_1$-$C_4$ alkyl-C(O)-O-, $C_1$-$C_4$ alkoxy-C(O)-O- or

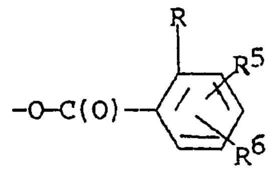

wherein R, $R^5$ and $R^6$ in this portion of the molecule are identical with those in the reactant shown above along with a mole amount or more of a moderate base to form an enol ester of the structural formula

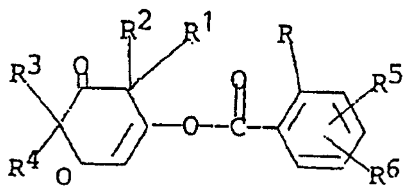

wherein R to $R^6$ are as defined and in step 2) reacting a mole amount of the enol ester intermediate with 1 to 4 moles of a moderate base and from 0.01 mole to 0.5 mole or higher of a cyanide source to form the desired herbicidal compound.

2. The process of Claim 1 wherein X is halogen, the moderate base is tri-$C_1$-$C_6$ alkylamine, pyridine, alkali metal carbonate or alkali metal phosphate and the cyanide source alkali metal cyanide, cyanohydrins of methyl $C_1$-$C_4$-alkyl ketones, cyanohydrins of benzaldehyde or $C_2$-$C_5$ aliphatic aldehydes; cyanohydrins, zinc cyanide; tri (lower alkyl) silyl cyanides or hydrogen cyanide.

3. The process of Claim 2 wherein X is chlorine, the molderate base is tri-$C_1$-$C_6$ alkylamine, pyridine, sodium carbonate or sodium phosphate and the cyanide source is potassium cyanide, acetone cyanohydrin or hydrogen cyanide.

4. The process of Claims 1, 2 or 3, characterized in that compounds are prepared wherein R is chlorine, hydrogen, bromine, methyl, methoxy, nitro, trifluoromethyl or methylsulfonyl; $R^1$, $R^2$, $R^3$ and $R^4$ are methyl; $R^5$ and $R^6$ independently are (1) hydrogen; (2) halogen; (3) $C_1$-$C_4$ alkyl; (4) $C_1$-$C_4$-alkoxy; (5) cyano; (6) nitro; (7) $C_1$-$C_4$ haloalkyl; (8) $R^bSO_n$- wherein n is the integer 0 or 2; and $R^b$ is (a) $C_1$-$C_4$ alkyl; (b) $C_1$-$C_4$ alkyl substituted with halogen; (9) 4-$SO_2N(CH_3)_2$.

5. The process of Claim 4, characterized in that compounds are prepared wherein $R^5$ is hydrogen and $R^6$ is hydrogen, chlorine, bromine, fluorine, cyano, trifluoromethyl or $R^bSO_2$ wherein $R^b$ is $C_1$-$C_4$ alkyl or $C_1$-$C_4$ haloalkyl.

6. The process of Claims 4 or 5, characterized in that compounds are prepared wherein $R^5$ is hydrogen.

7. The method of controlling undesirable vegetation comprising applying to the area where control is desired, an herbicidally effective amount of a compound as prepared according to anyone of the Claims 1 to 6.

8. A process for preparing an herbicidal composition comprising mixing an herbicidally active 2-benzoyl-substituted-1,3,5-cyclohexanetrione or their salts as prepared according to Claims 1 to 6 and an inert carrier therefor.

9. The method of controlling undesirable vegetation comprising applying to the area where control is desired, an herbicidal composition comprising an herbicidally active 2-benzoyl-substituted-1,3,5-cyclohexanetrione or its salt prepared according to anyone of the Claims 1 to 6 and an inert carried therefor.

## Patentansprüche

### Patentansprüche für folgende Vertragsstaaten : BE, CH, DE, FR, GB, GR, IT, LI und NL

1. Verbindungen der Formel:

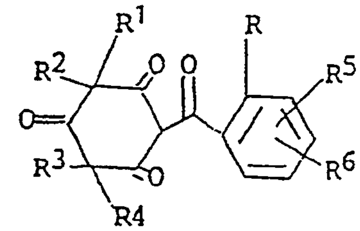

worin

R Wasserstoff, Nitro, Methyl, Methoxy, Trifluormethyl, Halogen oder $SO_2CH_3$ bedeutet;
$R^1$ Wasserstoff oder $C_1$-$C_4$-Alkyl bedeutet;
$R^2$ Wasserstoff oder $C_1$-$C_4$-Alkyl bedeutet; oder
$R^1$ und $R^2$ zusammen $C_2$-$C_5$-Alkylen bedeuten;
$R^3$ $C_1$-$C_4$-Alkyl bedeutet;
$R^4$ $C_1$-$C_4$-Alkyl bedeutet; oder worin
$R^3$ und $R^4$ zusammen $C_2$-$C_5$-Alkylen bedeuten;
$R^5$ Wasserstoff, 3-$C_1$-$C_4$-Alkoxy, 3-Nitro oder 3-Halogen bedeutet; und
$R^6$ Wasserstoff, 4-Halogen, 4-Trifluormethyl, 4-$R^bSO_n$, worin $R^b$ für $C_1$-$C_4$-Alkyl oder $C_1$-$C_4$-Haloalkyl steht und n die ganze Zahl 0 oder 2 ist, 4-Cyano, 4-C(O)$CH_3$, 4-$SCF_3$ oder 4-$SO_2N(CH_3)_2$ bedeutet,
und ihre Salze.

2. Verbindungen nach Anspruch 1, dadurch **gekennzeichnet**, daß R Chlor, Wasserstoff, Brom, Methyl, Methoxy, Nitro, Trifluormethyl oder Methylsulfonyl bedeutet; $R^1$, $R^2$, $R^3$ und $R^4$ Methyl bedeuten; $R^5$ und $R^6$ unabhängig (1) Wasserstoff; (2) Halogen; (3) $C_1$-$C_4$-Alkyl; (4) $C_1$-$C_4$-Alkoxy; (5) Cyano; (6) Nitro; (7) $C_1$-$C_4$-Haloalkyl; (8) $R^bSO_n$-, worin n die ganze Zahl 0 oder 2 ist und $R^b$ für (a) $C_1$-$C_4$-Alkyl; (b) $C_1$-$C_4$-Alkyl, substituiert mit Halogen, steht; (9) 4-$SO_2N(CH_3)_2$ bedeuten.

3. Verbindungen nach Anspruch 2, dadurch **gekennzeichnet**, daß $R^5$ Wasserstoff bedeutet und $R^6$ Wasserstoff, Chlor, Brom, Fluor, Cyano, Trifluormethyl oder $R^bSO_2$, worin $R^b$ für $C_1$-$C_4$-Alkyl oder $C_1$-$C_4$-Haloalkyl steht, bedeutet.

4. Verbindungen nach Anspruch 2 oder 3, dadurch **gekennzeichnet**, daß $R^5$ Wasserstoff bedeutet.

5. Verfahren zur Kontrolle unerwünschter Vegetation, dadurch **gekennzeichnet**, daß man auf die Fläche, wo die Kontrolle erfolgen soll, eine herbizid wirksame Menge einer Verbindung nach einem der Ansprüche 1, 2, 3 oder 4 anwendet.

6. Herbizides Mittel, dadurch **gekennzeichnet**, daß es ein herbizid aktives 2-Benzoyl-substituiertes 1,3,5-Cyclohexantrion oder seine Salze, wie in den Ansprüchen 1, 2, 3 oder 4 beschrieben, und einen inerten Träger dafür enthält.

7. Verfahren zur Kontrolle unerwünschter Vegetation, dadurch **gekennzeichnet**, daß auf die Fläche, wo eine kontrolle erfolgen soll, ein herbizides Mittel angewendet wird, das ein herbizid aktives 2-Benzoyl-substituiertes 1,3,5-Cyclohexantrion oder sein Salz, wie in Anspruch 6 beschrieben, und einen inerten Träger dafür enthält.

8. Zwischenprodukt der Strukturformel:

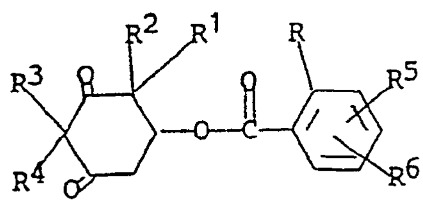

worin

R Wasserstoff, Nitro, Methyl, Methoxy, Trifluormethyl, Halogen oder $SO_2CH_3$ bedeutet;

$R^1$ Wasserstoff oder $C_1$-$C_4$-Alkyl bedeutet;
$R^2$ Wasserstoff oder $C_1$-$C_4$-Alkyl bedeutet; oder
$R^1$ und $R^2$ zusammen $C_2$-$C_5$-Alkylen bedeuten;
$R^3$ $C_1$-$C_4$-Alkyl bedeutet;
$R^4$ $C_1$-$C_4$-Alkyl bedeutet; oder worin
$R^3$ und $R^4$ zusammen $C_2$-$C_5$-Alkylen bedeuten;
$R^5$ Wasserstoff, 3-$C_1$-$C_4$-Alkoxy, 3-Nitro oder 3-Halogen bedeutet; und
$R^6$ Wasserstoff, 4-Halogen, 4-Trifluormethyl, 4-$R^bSO_n$, worin $R^b$ für $C_1$-$C_4$-Alkyl oder $C_1$-$C_4$-Haloalkyl steht und n die ganze Zahl 0 oder 2 ist, 4-Cyano, 4-C(O)$CH_3$, 4-$SCF_3$ oder 4-$SO_2N(CH_3)_2$ bedeutet.

9. Verfahren zur Herstellung einer herbiziden Verbindung der Strukturformel:

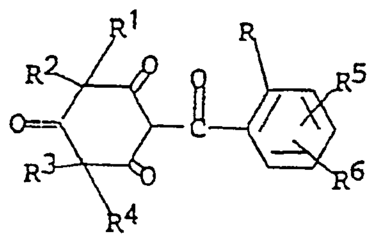

worin

R Wasserstoff, Nitro, Methyl, Methoxy, Trifluormethyl, Halogen oder $SO_2CH_3$ bedeutet;
$R^1$ Wasserstoff oder $C_1$-$C_4$-Alkyl bedeutet;
$R^2$ Wasserstoff oder $C_1$-$C_4$-Alkyl bedeutet; oder
$R^1$ und $R^2$ zusammen $C_2$-$C_5$-Alkylen bedeuten;
$R^3$ $C_1$-$C_4$-Alkyl bedeutet;
$R^4$ $C_1$-$C_4$-Alkyl bedeutet; oder worin
$R^3$ und $R^4$ zusammen $C_2$-$C_5$-Alkylen bedeuten;
$R^5$ Wasserstoff, 3-$C_1$-$C_4$-Alkoxy, 3-Nitro oder 3-Halogen bedeutet; und
$R^6$ Wasserstoff, 4-Halogen, 4-Trifluormethyl, 4-$R^bSO_n$, worin $R^b$ für $C_1$-$C_4$-Alkyl oder $C_1$-$C_4$-Haloalkyl steht und n die ganze Zahl 0 oder 2 ist, 4-Cyano, 4-C(O)$CH_3$, 4-$SCF_3$ oder 4-$SO_2N(CH_3)_2$ bedeutet,
und ihrer Salze,

**gekennzeichnet** durch die Stufen 1) Umsetzung einer Molmenge eines Trions der Strukturformel:

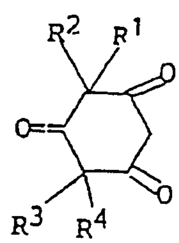

worin $R^1$ bis $R^4$ die gegebenen Definitionen besitzen, mit einer Molmenge eines substituierten Benzoyl-Reaktionsteilnehmers der Strukturformel:

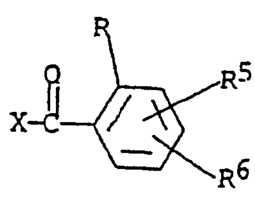

worin R, $R^5$ und $R^6$ die gegebenen Definitionen besitzen und X Halogen, $C_1$-$C_4$-Alkyl-C(O)-O-, $C_1$-$C_4$-Alkoxy-C(O)-O- oder

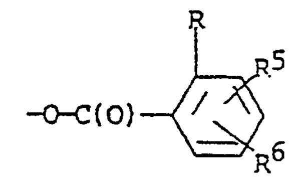

bedeutet, worin R, $R^5$ und $R^6$ in diesem Teil des Moleküls identisch sind mit jenem in dem oben gezeigten Reaktionsteilnehmer, zusammen mit einer Molmenge oder mehr einer milden Base unter Bildung eines Enolesters der Strukturformel:

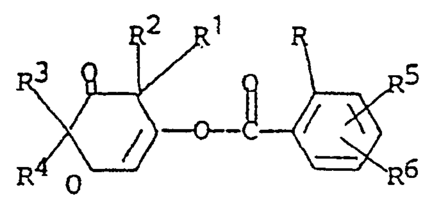

worin R bis $R^6$ die gegebenen Definitionen besitzen, und bei der Stufe 2) Umsetzung einer Molmenge des Enolester-Zwischenprodukts mit 1 bis 4 mol einer milden Base und von 0,01 mol bis 0,5 mol oder mehr einer Cyanidquelle unter Bildung der gewünschten herbiziden Verbindung.

10. Verfahren nach Anspruch 9, dadurch **gekennzeichnet**, daß X Halogen bedeutet, die milde Base Tri-$C_1$-$C_6$-alkylamin, Pyridin, ein Alkalimetallcarbonat oder ein Alkalimetallphosphat ist und die Cyanidquelle ein Alkalimetallcyanid, Cyanohydrine von Methyl-$C_1$-$C_4$-alkylketonen, Cyanohydrine von Benzaldehyd oder $C_2$-$C_5$-aliphatischen Aldehyden, Cyanohydrine, Zinkcyanid, Tri-(niedrigalkyl)-silylcyanide oder Cyanwasserstoff ist.

11. Verfahren nach Anspruch 10, dadurch **gekennzeichnet**, daß X Chlor bedeutet, die milde Base Tri-$C_1$-$C_6$-alkylamin, Pyridin, Natriumcarbonat oder Natriumphosphat ist und die Cyanidquelle Kaliumcyanid, Acetoncyanohydrin oder Cyanwasserstoff ist.

**Patentansprüche für folgende Vertragsstaaten : AT und ES**

1. Verfahren zur Herstellung einer herbiziden Verbindung der Strukturformel:

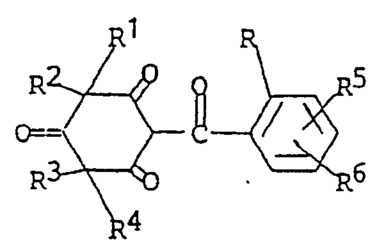

worin

R Wasserstoff, Nitro, Methyl, Methoxy, Trifluormethyl, Halogen oder $SO_2CH_3$ bedeutet;
$R^1$ Wasserstoff oder $C_1$-$C_4$-Alkyl bedeutet;
$R^2$ Wasserstoff oder $C_1$-$C_4$-Alkyl bedeutet; oder
$R^1$ und $R^2$ zusammen $C_2$-$C_5$-Alkylen bedeuten;
$R^3$ $C_1$-$C_4$-Alkyl bedeutet;
$R^4$ $C_1$-$C_4$-Alkyl bedeutet; oder worin
$R^3$ und $R^4$ zusammen $C_2$-$C_5$-Alkylen bedeuten;
$R^5$ Wasserstoff, 3-$C_1$-$C_4$-Alkoxy, 3-Nitro oder 3-Halogen bedeutet; und
$R^6$ Wasserstoff, 4-Halogen, 4-Trifluormethyl, 4-$R^bSO_n$, worin $R^b$ für $C_1$-$C_4$-Alkyl oder $C_1$-$C_4$-Haloalkyl steht und n die ganze Zahl 0 oder 2 ist, 4-Cyano, 4-$C(O)CH_3$, 4-$SCF_3$ oder 4-$SO_2N(CH_3)_2$ bedeutet,

und ihrer Salze,

**gekennzeichnet** durch die Stufen 1) Umsetzung einer Molmenge eines Trions der Strukturformel:

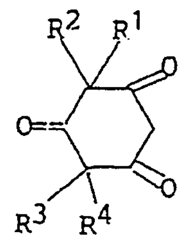

worin $R^1$ bis $R^4$ die gegebenen Definitionen besitzen, mit einer Molmenge eines substituierten Benzoyl-Reaktionsteilnehmers der Strukturformel:

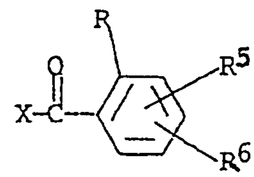

worin R, $R^5$ und $R^6$ die gegebenen Definitionen besitzen und X Halogen, $C_1$-$C_4$-Alkyl-C(O)-O-, $C_1$-$C_4$-Alkoxy-C(O)-O- oder

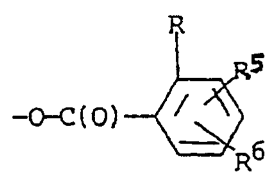

bedeutet, worin R, $R^5$ und $R^6$ in diesem Teil des Moleküls identisch sind mit jenem in dem oben gezeigten Reaktionsteilnehmer, zusammen mit einer Molmenge oder mehr einer milden Base unter Bildung eines Enolesters der Strukturformel:

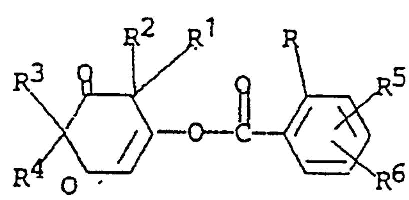

worin R bis $R^6$ die gegebenen Definitionen besitzen, und bei der Stufe 2) Umsetzung einer Molmenge des Enolester-Zwischenprodukts mit 1 bis 4 mol einer milden Base und von 0,01 mol bis 0,5 mol oder mehr einer Cyanidquelle unter Bildung der gewünschten herbiziden Verbindung.

2. Verfahren nach Anspruch 1, dadurch **gekennzeichnet**, daß X halogen bedeutet, die milde Base Tri-$C_1$-$C_6$-alkylamin, Pyridin, ein Alkalimetallcarbonat oder ein Alkalimetallphosphat ist und die Cyanidquelle ein Alkalimetallcyanid, Cyanohydrine von Methyl-$C_1$-$C_4$-alkylketonen, Cyanohydrine von Benzaldehyd oder $C_2$-$C_5$-aliphatischen Aldehyden, Cyanohydrine, Zinkcyanid, Tri-(niedrigalkyl)-silylcyanide oder Cyanwasserstoff ist.

3. Verfahren nach Anspruch 2, dadurch **gekennzeichnet**, daß X Chlor bedeutet, die milde Base Tri-$C_1$-$C_6$-alkylamin, Pyridin, Natriumcarbonat oder Natriumphosphat ist und die Cyanidquelle Kaliumcyanid, Acetoncyanohydrin oder Cyanwasserstoff ist.

4. Verfahren nach einem der Ansprüche 1, 2 oder 3, dadurch **gekennzeichnet**, daß Verbindungen hergestellt werden, worin R Chlor, Wasserstoff, Brom, Methyl, Methoxy, Nitro, Trifluormethyl oder Methylsulfonyl bedeutet; $R^1$, $R^2$, $R^3$ und $R^4$ Methyl bedeuten; $R^5$ und $R^6$ unabhängig (1) Wasserstoff; (2) Halogen; (3) $C_1$-$C_4$-Alkyl; (4) $C_1$-$C_4$-Alkoxy; (5) Cyano; (6) Nitro; (7) $C_1$-$C_4$-Haloalkyl; (8) $R^bSO_n$-, worin n die ganze Zahl 0 oder 2 ist und $R^b$ für (a) $C_1$-$C_4$-Alkyl; (b) $C_1$-$C_4$-Alkyl, substituiert mit Halogen, steht; (9) 4-$SO_2N(CH_3)_2$ bedeuten.

5. Verfahren nach Anspruch 4, dadurch **gekennzeichnet**, daß Verbindungen hergestellt werden, worin $R^5$

Wasserstoff bedeutet und $R^6$ Wasserstoff, Chlor, Brom, Fluor, Cyano, Trifluormethyl oder $R^bSO_2$, worin $R^b$ für $C_1$-$C_4$-Alkyl oder $C_1$-$C_4$-Haloalkyl steht, bedeutet.

6. Verfahren nach Anspruch 4 oder 5, dadurch **gekennzeichnet**, daß Verbindungen hergestellt werden, worin $R^5$ Wasserstoff bedeutet.

7. Verfahren zur Kontrolle unerwünschter Vegetation, dadurch **gekennzeichnet**, daß man auf die Fläche, wo die Kontrolle erfolgen soll, eine herbizid wirksame Menge einer Verbindung, hergestellt nach einem der Ansprüche 1 bis 6, anwendet.

8. Verfahren zur Herstellung eines herbiziden Mittels, dadurch **gekennzeichnet**, daß ein herbizid aktives 2-Benzoyl-substituiertes 1,3,5-Cyclohexantrion oder seine Salze, hergestellt nach einem der Ansprüche 1 bis 6, und einen inerter Träger dafür vermischt werden.

9. Verfahren zur Kontrolle unerwünschter Vegetation, dadurch **gekennzeichnet**, daß auf die Fläche, wo eine Kontrolle erfolgen soll, ein herbizides Mittel angewendet wird, das ein herbizid aktives 2-Benzoyl-substituiertes 1,3,5-Cyclohexantrion oder sein Salz, hergestellt nach einem der Ansprüche 1 bis 6, und einen inerten Träger dafür enthält.

## Revendications

### Revendications pour les Etats contractants suivants : BE, CH, DE, FR, GB, GR, IT, LI, NL

1. Composés représentés par la formule :

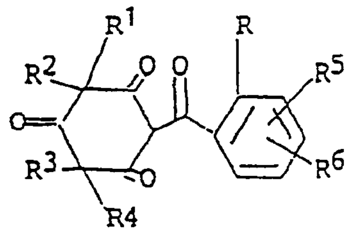

dans laquelle

R est un atome d'hydrogène, un groupe nitro, méthyle, méthoxy, trifluorométhyle, un atome d'halogène ou un radical $SO_2CH_3$; et

$R^1$ est un atome d'hydrogène ou un groupe alkyle en $C_{1-4}$;

$R^2$ est un atome d'hydrogène ou un groupe alkyle en $C_{1-4}$; ou

$R^1$ et $R^2$ ensemble sont un groupe alkylène en $C_{2-5}$;

$R^3$ est un groupe alkyle en $C_{1-4}$;

$R^4$ est un groupe alkyle en $C_{1-4}$; ou bien

$R^3$ et $R^4$ ensemble sont un groupe alkylène en $C_{2-5}$;

$R^5$ est un atome d'hydrogène, un groupe 3-alcoxy en $C_{1-4}$, un groupe 3-nitro ou un atome d'halogène en position 3; et

$R^6$ est un atome d'hydrogène, un atome d'halogène en position 4, un groupe 4-trifluorométhyle, 4-$R^bSO_n$ dans lequel $R^b$ est un groupe alkyle en $C_{1-4}$ ou haloalkyle en $C_{1-4}$ et n est l'entier 0 ou 2,4-cyano, 4-C(O)$CH_3$, 4-$SCF_3$ ou 4-$SO_2N(CH_3)_2$,

et leurs sels.

2. Les composés suivant la revendication 1, dans lesquels R est un atome d'hydrogène, de chlore ou de brome, un groupe méthyle, méthoxy, nitro, trifluorométhyle ou méthylsulfonyle; $R^1$, $R^2$, $R^3$ et $R^4$ sont chacun un groupe méthyle; $R^5$ et $R^6$ sont indépendamment (1) un atome d'hydrogène, (2) un atome d'halogène, (3) un groupe alkyle en $C_{1-4}$; (4) un groupe alcoxy en $C_{1-4}$, (5) un groupe cyano, (6) un groupe nitro, (7) un groupe haloalkyle en $C_{1-4}$, (8) un radical $R^bSO_n$ dans lequel n est l'entier 0 ou 2, et $R^b$ est (a) un groupe alkyle en $C_{1-4}$, (b) un groupe alkyle en $C_{1-4}$ halosubstitué; et (9) un radical 4-$SO_2N(CH_3)_2$.

3. Les composés suivant la revendication 2, dans lesquels $R^5$ est un atome d'hydrogène et $R^6$ est un atome d'hydrogène, de chlore, de brome, de fluor, un groupe cyano, trifluorométhyle ou $R^bSO_2$ dans lequel $R^b$ est un groupe alkyle en $C_{1-4}$, ou haloalkyle en $C_{1-4}$.

4. Les composés suivant les revendications 2 ou 3, dans lesquels $R^5$ est un atome d'hydrogène.

5. Le procédé pour contrôler une végétation indésirable, qui comprend l'application à la surface dans

laquelle un contrôle est désiré, d'une quantité efficace du point de vue herbicide d'un composé suivant l'une quelconque des revendications 1, 2, 3 ou 4.

6. Une composition herbicide comprenant une 2-benzoyl-1,3,5-cyclohexanetrione substituée active du point de vue herbicide ou ses sels suivant l'une quelconque des revendications 1, 2, 3 ou 4 et un support inerte pour ceux-ci.

7. Le procédé pour contrôler une végétation indésirable, qui comprend l'application à la surface dans laquelle un contrôle est désiré, d'une quantité efficace du point de vue herbicide d'une 2-benzoyl-1,3,5-cyclohexanetrione substituée active du point de vue herbicide ou de son sel suivant la revendication 6 et un support inerte pour ceux-ci.

8. Un composé intermédiaire de formule structurale :

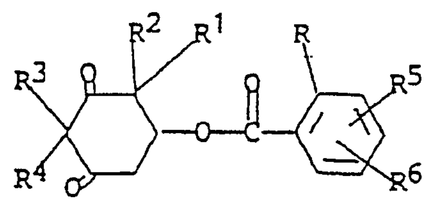

dans laquelle

R est un atome d'hydrogène, un groupe nitro, méthyle, méthoxy, trifluorométhyle, un atome d'halogène ou un radical $SO_2CH_3$; et

$R^1$ est un atome d'hydrogène ou un groupe alkyle en $C_{1-4}$;

$R^2$ est un atome d'hydrogène ou un groupe alkyle en $C_{1-4}$; ou

$R^1$ et $R^2$ ensemble sont un groupe alkylène en $C_{2-5}$;

$R^3$ est un groupe alkyle en $C_{1-4}$;

$R^4$ est un groupe alkyle en $C_{1-4}$; ou bien

$R^3$ et $R^4$ ensemble sont un groupe alkylène en $C_{2-5}$;

$R^5$ est un atome d'hydrogène, un groupe 3-alcoxy en $C_{1-4}$, un groupe 3-nitro ou un atome d'halogène en position 3; et

$R^6$ est un atome d'hydrogène, un atome d'halogène en position 4, un groupe 4-trifluorométhyle, 4-$R^bSO_n$ dans lequel $R^b$ est un groupe alkyle en $C_{1-4}$ ou haloalkyle en $C_{1-4}$ et n est l'entier 0 ou 2,4-cyano, 4-$C(O)CH_3$, 4-$SCF_3$ ou 4-$SO_2N(CH_3)_2$.

9. Un procédé pour préparer un composé herbicide de formule structurale :

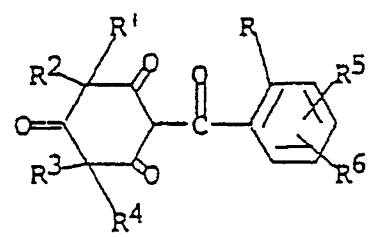

dans laquelle

R est un atome d'hydrogène, un groupe nitro, méthyle, méthoxy, trifluorométhyle, un atome d'halogène ou un radical $SO_2CH_3$; et

$R^1$ est un atome d'hydrogène ou un groupe alkyle en $C_{1-4}$;

$R^2$ est un atome d'hydrogène ou un groupe alkyle en $C_{1-4}$; ou

$R^1$ et $R^2$ ensemble sont un groupe alkylène en $C_{2-5}$;

$R^3$ est un groupe alkyle en $C_{1-4}$;

$R^4$ est un groupe alkyle en $C_{1-4}$; ou bien

$R^3$ et $R^4$ ensemble sont un groupe alkylène en $C_{2-5}$;

$R^5$ est un atome d'hydrogène, un groupe 3-alcoxy en $C_{1-4}$, un groupe 3-nitro ou un atome d'halogène en position 3; et

$R^6$ est un atome d'hydrogène, un atome d'halogène en position 4, un groupe 4-trifluorométhyle, 4-$R^bSO_n$ dans lequel $R^b$ est un groupe alkyle en $C_{1-4}$ ou haloalkyle en $C_{1-4}$ et n est l'entier 0 ou 2,4-cyano, 4-$C(O)CH_3$,

$4-SCF_3$ ou $4-SO_2N(CH_3)_2$,
et ses sels,
comprenant les étapes suivantes : (1) réaction d'une quantité molaire d'une trione de formule structurale

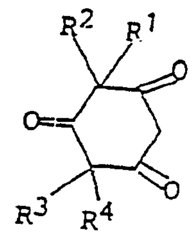

dans laquelle $R^1$ à $R^4$ sont tels que définis, avec une quantité molaire d'un réactif benzoyle substitué de formule structurale :

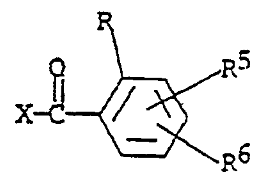

dans laquelle R, $R^5$ et $R^6$ sont tels que définis plus haut, X est un atome d'halogène, un groupe alkyl(en $C_{1-4}$)-C(O)-O-, un groupe alcoxy(en $C_{1-4}$)-C(O)-O- ou bien

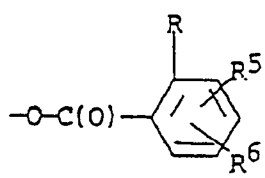

dans laquelle R, $R^5$ et $R^6$ dans cette partie de la molécule sont identiques à ceux du réactif montré ci-dessus, avec une quantité molaire ou supérieur d'une base modérée pour former un énol ester de formule structurale :

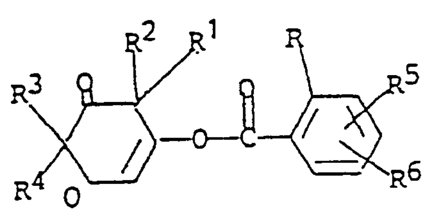

dans laquelle R à $R^6$ sont tels que définis et (2) réaction d'une quantité molaire de l'énol ester intermédiaire avec 1 à 4 moles d'une base modérée et de 0,01 à 0,5 mole ou plus, d'une source de cyanure pour former le composé herbicide désiré.

10. Le procédé suivant la revendication 9, dans lequel X est un atome d'halogène, la base modérée est une trialkyl-(en $C_{1-6}$)-amine, la pyridine, un carbonate de métal alcalin ou un phosphate de métal alcalin et la source de cyanure est un cyanure de métal alcalin, des cyanhydrines de méthyl alkyl(en $C_{1-4}$) cétone, des cyanhydrines de benzaldéhyde ou d'aldéhydes aliphatiques en $C_{2-5}$; des cyanhydrines, le cyanure de zinc, des cyanures de tri(alkyle inférieur)silyle ou le cyanure d'hydrogène.

11. Le procédé suivant la revendication 10, dans lequel X est un atome de chlore, la base modérée est une trialkyl-(en $C_{1-6}$)-amine, la pyridine, le carbonate de sodium ou le phosphate de sodium et la source de cyanure est le cyanure de potassium, l'acétone cyanhydrine ou le cyanure d'hydrogène.

**Revendications pour les Etats contractants suivants : AT et ES**

1. Un procédé de préparation d'un composé herbicide de formule structurale :

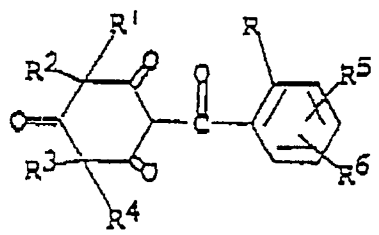

dans laquelle

R est un atome d'hydrogène, un groupe nitro, méthyle, méthoxy, trifluorométhyle, un atome d'halogène ou un radical $SO_2CH_3$; et

$R^1$ est un atome d'hydrogène ou un groupe alkyle en $C_{1-4}$;

$R^2$ est un atome d'hydrogène ou un groupe alkyle en $C_{1-4}$; ou

$R^1$ et $R^2$ ensemble sont un groupe alkylène en $C_{2-5}$;

$R^3$ est un groupe alkyle en $C_{1-4}$;

$R^4$ est un groupe alkyle en $C_{1-4}$; ou bien

$R^3$ et $R^4$ ensemble sont un groupe alkylène en $C_{2-5}$;

$R^5$ est un atome d'hydrogène, un groupe 3-alcoxy en $C_{1-4}$, un groupe 3-nitro ou un atome d'halogène en position 3; et

$R^6$ est un atome d'hydrogène, un atome d'halogène en position 4, un groupe 4-trifluorométhyle, 4-$R^bSO_n$ dans lesquel $R^b$ est un groupe alkyle en $C_{1-4}$ ou haloalkyle en $C_{1-4}$ et n est l'entier 0 ou 2,4-cyano, 4-$C(O)CH_3$, 4-$SCF_3$ ou 4-$SO_2N(CH_3)_2$,

et ses sels,

comprenant les étapes suivantes : (1) réaction d'une quantité molaire d'une trione de formule structurale

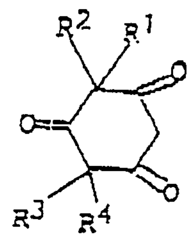

dans laquelle $R^1$ à $R^4$ sont tels que définis, avec une quantité molaire d'un réactif benzoyle substitué de formule structurale :

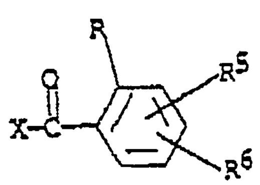

dans laquelle R, $R^5$ et $R^6$ sont tels que définis plus haut, X est un atome d'halogène, un groupe alkyl(en $C_{1-4}$)-C(O)-O-, un groupe alcoxy(en $C_{1-4}$)-C(O)-O- ou bien

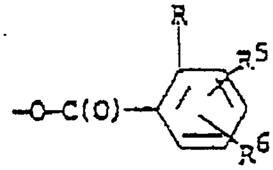

dans laquelle R, $R^5$ et $R^6$ dans cette partie de la molécule sont identiques à ceux du réactif montré ci-dessus, avec une quantité molaire ou supérieure d'une base modérée pour former un énol ester de formule structurale :

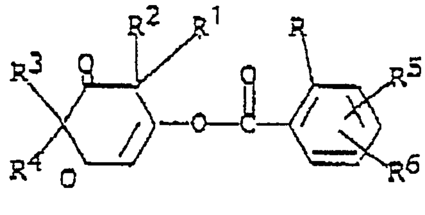

dans laquelle R à $R^6$ sont tels que définis et (2) réaction d'une quantité molaire de l'énol ester intermédiaire avec 1 à 4 moles d'une base modérée et de 0,01 à 0,5 mole ou plus, d'une source de cyanure pour former le composé herbicide désiré.

2. Le procédé suivant la revendication 1, caractérisé en ce que, dans les composés préparés, X est un atome d'halogène, la base modérée est une trialkyl-(en $C_{1-6}$)-amine, la pyridine, un carbonate de métal alcalin ou un phosphate de métal alcalin et la source de cyanure est un cyanure de métal alcalin, des cyanhydrines de méthyl alkyl(en $C_{1-4}$) cétone, des cyanhydrines de benzaldéhyde ou d'aldéhydes aliphatiques en $C_{2-5}$; des cyanhydrines, le cyanure de zinc, des cyanures de tri(alkyle inférieur)silyle ou le cyanure d'hydrogène.

3. Le procédé suivant la revendication 2, caractérisé en ce que, dans les composés préparés, X est un atome de chlore, la base modérée est une trialkyl-(en $C_{1-6}$)-amine, la pyridine, le carbonate de sodium ou le phosphate de sodium et la source de cyanure est le cyanure de potassium, l'acétone cyanhydrine ou le cyanure d'hydrogène.

4. Le procédé suivant l'une quelconque des revendications 1, 2 et 3, caractérisé en ce que, dans les composés préparés, R est un atome d'hydrogène, de chlore ou de brome, un groupe méthyle, méthoxy, nitro, trifluorométhyle ou méthylsulfonyle; $R^1$, $R^2$, $R^3$ et $R^4$ sont chacun un groupe méthyle; $R^5$ et $R^6$ sont indépendamment (1) un atome d'hydrogène, (2) un atome d'halogène, (3) un groupe alkyle en $C_{1-4}$; (4) un groupe alcoxy en $C_{1-4}$, (5) un groupe cyano, (6) un groupe nitro, (7) un groupe haloalkyle en $C_{1-4}$, (8) un radical $R^bSO_n$ dans lequel n est l'entier 0 ou 2, et $R^b$ est (a) un groupe alkyle en $C_{1-4}$, (b) un groupe alkyle en $C_{1-4}$ halosubstitué; et (9) un radical 4-$SO_2N(CH_3)_2$.

5. Le procédé suivant la revendication 4, caractérisé en ce que, dans les composés préparés, $R^5$ est un atome d'hydrogène et $R^6$ est un atome d'hydrogène, de chlore, de brome, de fluor, un groupe cyano, trifluorométhyle ou $R^bSO_n$ dans lequel $R^b$ est un groupe alkyle en $C_{1-4}$, ou haloalkyle en $C_{1-4}$.

6. Le procédé suivant les revendications 4 ou 5, caractérisé en ce que, dans les composés préparés, $R^5$ est un atome d'hydrogène.

7. Le procédé pour contrôler une végétation indésirable, caractérisé en ce qu'il comprend l'application à la surface dans laquelle un contrôle est désiré, d'une quantité efficace du point de vue herbicide d'un composé préparé par le procédé suivant l'une quelconque des revendirations 1 à 6.

8. Un procédé de préparation d'une composition herbicide caractérisé en ce qu'il comprend le mélange d'une 2-benzoyl-1,3,5-cyclohexanetrione substituée active du point de vue herbicide ou ses sels préparés par le procédé suivant l'une quelconque des revendications 1 à 6 et d'un support inerte pour ceux-ci.

9. Le procédé pour contrôler une végétation indésirable, caractérisé en ce qu'il comprend l'application à la surface dans laquelle un contrôle est désiré, d'une quantité efficace du point de vue herbicide d'une 2-benzoyl-1,3,5-cyclohexanetrione substituée active du point de vue herbicide ou de son sel préparés par le procédé suivant l'une quelconque des revendication 1 à 6 et un support inerte pour ceux-ci.